# EUROPEAN PATENT SPECIFICATION

(11) **EP 4 337 414 B1**
(45) Date of publication and mention of the grant of the patent: **17.06.2026**
(21) Application number: 22808120.4
(22) Date of filing: 09.05.2022
(51) Int. Cl.: B23P 6/00, F28D 7/00, F28F 11/00, F28D 7/16, F28D 21/00, F28F 27/00, F28G 15/00

(54) **METHOD FOR MONITORING A TUBE SHEET OF A HEAT EXCHANGER**
VERFAHREN ZUR ÜBERWACHUNG EINES ROHRBODENS EINES WÄRMETAUSCHERS
PROCÉDÉ DE SURVEILLANCE DE PLAQUE TUBULAIRE D'ÉCHANGEUR DE CHALEUR

(30) Priority: 11.05.2021 US 202163186932 P
(43) Date of publication of application: 20.03.2024
(73) Proprietor: Arkema Inc., King of Prussia, PA 19406 (US)
(72) Inventor: DECOURCY, Michael, S., King of Prussia, Pennsylvania 19406 (US); TRIPATHY, Kishlay, King of Prussia, Pennsylvania 19406 (US)
(74) Representative: Arkema Patent
(86) International application number: PCT/US2022/028307
(87) International publication number: WO 2022/240737

(56) References cited:
- WO-A1-2015/002451
- KR-B1- 101 245 548
- US-A1- 2009 112 367
- US-A1- 2012 097 378
- US-A1- 2020 003 503
- US-B2- 8 485 139
- PANAHI H.; ESLAMI A.; GOLOZAR M.A.; ASHRAFI LALEH A.: "An investigation on corrosion failure of a shell-and-tube heat exchanger in a natural gas treating plant", ENGINEERING FAILURE ANALYSIS, PERGAMON, GB, vol. 118, 14 September 2020 (2020-09-14), GB , XP086319039, ISSN: 1350-6307, DOI: 10.1016/j.engfailanal.2020.104918
- LIU LONG, DING NING, SHI JUNBO, XU NA, GUO WEIMIN, WU CHI-MAN LAWRENCE: "Failure analysis of tube-to-tubesheet welded joints in a shell-tube heat exchanger", CASE STUDIES IN ENGINEERING FAILURE ANALYSIS, vol. 7, 1 October 2016 (2016-10-01), pages 32 - 40, XP093014324, ISSN: 2213-2902, DOI: 10.1016/j.csefa.2016.06.002

## Description

### FIELD OF THE INVENTION

The invention relates to a method for monitoring a tube sheet of a heat exchanger during a maintenance activity.

### BACKGROUND OF THE INVENTION

Document KR 10-1245548 describes a camera for boiler tube shooting, and a cowling monitoring system to calculate a degree of cowling formation in a tube.

Document US Patent No. 8,485,139 describes an apparatus for visually inspecting a structure including a heating tube, a tube sheet supporting the heating tube, and a flow distribution baffle, which are installed in a steam generator of a nuclear power plant, and removing a foreign object in gaps of a bundle of heating tubes of a tube sheet.

Neither of the cited references teaches or suggests acquiring a pair of initial digital images from two different vantage points.

Shell-and-tube devices, such as heat exchangers, can comprise hundreds or thousands of tubes. Shell-and-tube heat exchangers typically require regular maintenance, such as cleaning and inspection of the individual tubes, to assure reliability and safe operation. Further, shell-and-tube reactors require regular catalyst replacement for optimal productivity. Due to the large number of tubes present, maintenance activities require significant manpower expense and extended periods of process downtime to complete; thus, there is a strong economic incentive to perform these activities quickly and efficiently. Failing to properly perform maintenance activities on every tube within a shell-and-tube exchanger can lead to costly process downtime, equipment damage, and shortened catalyst service life within reactors.

### SUMMARY OF THE INVENTION

Described herein is an automated method for tracking the status of individual tubes of a shell and tube device during maintenance activities and recording status data for review and analysis. In particular, the method monitors for the performance of a "projection-type" maintenance activity within a shell and tube device, wherein the appearance of an object projecting out from an individual tube is tracked. This is a subset of the larger group of all maintenance activities. When a projecting object appears within the field of view of an imaging device(s), it indicates that a specific maintenance activity, such as emptying, cleaning or inspecting, has been performed on a given tube. After detecting the projecting object, the method further comprises determining the unique identifier of the tube from which the object is projecting.

The utility of this monitoring method is that it will identify omission errors (tubes for which the maintenance activity was not performed) and it will also identify performance errors, such as when (i) a cleaning projectile becomes lodged in the tube; (ii) a cleaning device does not clean the entire length of the tube; or (iii) a fishtape does not fully empty catalyst from the tube. Additionally, this method can also identify repeat projections in which the monitored activity is performed on the same tube multiple times - which is undesirable.

The present method may be performed with one pair of imaging devices (e.g., cameras, non-contact ranging devices (NRD's)) positioned such that they are capable of viewing the same portion of the tubesheet surface from different vantage points. In practice, this means the imaging devices are placed near and at some elevation above the plane of the tubesheet, and adjusted to simultaneously view the tubesheet from two different sides.

Because the viewing angle for any imaging device may not be perpendicular to the tube sheet, and generally the projecting object being tracked does not consistently extend straight out of each tube (rather, it is often at an angle relative to the center line of the tube), the attributes of more than one tube within the image will be affected. Given that the projecting object is actually passing through only one tube, changes in the attributes of multiple tubes can become a complicating factor.

One method of the present invention therefore uses two views and array mathematics to determine which individual tube the projecting object is either passing through or exiting. This may be accomplished, for example, through the use of one pair of imaging devices.

In order to simplify image processing, however, it may be preferred that both imaging devices in the pair be of the same type (e.g., both LiDAR devices). More than two imaging devices may be used in order to address field of view (FOV) limitations (allowing for the creation of "mosaic" digital images, for example), but only two imaging devices may be required in order to perform the image-processing steps of the method. Alternatively, only one imaging device may be used.

Another method of the present invention uses at least two views and array mathematics to create an enhanced digital image. Enhanced digital images may be beneficial when processing blurred or distorted images comprising fast-moving objects, such as projectiles. The method utilizes the enhanced digital image to determine which individual tube the projecting object is either passing through or exiting.

Furthermore, status data may optionally be reported in real-time summary format and/or used to predict time-to-completion of a maintenance activity. The described method minimizes omission and performance errors and helps to reduce the expense of performing maintenance activities in shell-and-tube devices including heat exchangers and reactors.

### BRIEF DESCRIPTION OF THE DRAWING FIGURES

FIG. 1 depicts a system for monitoring a shell and tube device.
FIG. 2A depicts an exemplary embodiment of a horizontally oriented shell and tube heat exchanger.
FIG. 2B depicts one of the tube sheets of the heat exchanger of FIG. 2A.
FIG. 3A depicts another exemplary embodiment of a vertically oriented shell and tube heat exchanger.
FIG. 3B depicts one of the tube sheets of the heat exchanger of FIG. 3A.
FIG. 4 depicts an isometric schematic view of optical devices acquiring images of a tube sheet.
FIG. 5 depicts a schematic representation of the views acquired by the optical devices in FIG. 4.
FIG. 6 depicts a plan schematic view of the optical devices acquiring images of the tube sheet that is shown in FIG. 4.
FIG. 7 depicts a schematic illustration of the image capture and data collection process.
FIG. 8 depicts a real-time display of a maintenance activity (fishtaping).

### DETAILED DESCRIPTION OF THE INVENTION

### (A) System for Monitoring Shell and Tube Device

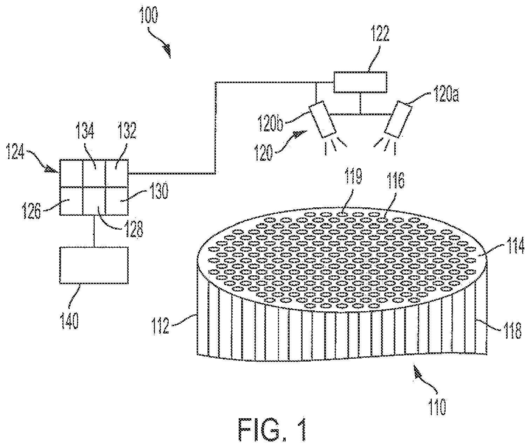

FIG. 1 depicts a system 100 for monitoring a shell and tube device 110. Shell and tube device 110, which does not necessarily form part of system 100, comprises a hollow shell 112 (a portion of which is shown) including a tube sheet 114 mounted to an end thereof. Tube sheet 114 has a series of holes 116 defined therethrough. Tubes 118 are mounted to respective holes 116 and positioned within hollow shell 112. Shell 112 is shown cut away to reveal the tubes 118. Ends 119 of the tubes 118 are exposed through the holes 116. The tubes 118 and their respective holes/passages may be circular, as shown, square, rectangular, and so forth.

System 100 generally comprises an imaging system 120 that is positioned above and/or beside holes 116. Imaging system 120 is configured for viewing holes 116. As will be described in greater detail below, imaging system 120 may comprise one imaging device 120a (a camera, for example). Alternatively, imaging system 120 may comprise multiple imaging devices 120a and 120b, for viewing holes 116 at different angles and vantage points. Imaging devices 120a and 120b are positioned at some elevation above the top plane of tube sheet 114. Imaging devices 120a and 120b may be stationary. Alternatively, one or more imaging devices may be mounted to a mobile device 122, such as an X-Y-Z translation stage, X-Y translation stage, or vehicle.

Imaging system 120 is configured to communicate data relating to maintenance activity at the tube ends 119 to a computer 124. Computer 124 may include an image processor 126, memory 128, clock 130, programming software 132, and a relational database 134 (among other features). Processor 126 is configured to analyze the data related to the tube ends 119, as will be described below. Computer 124 is connected to a display 140 for displaying the analyzed data, as will also be described below. Interconnections between display 140, imaging system 120 and computer 124 may be either wired or wireless, for example.

Further details and alternative features in connection with system 100 and device 110 are provided hereinafter.

### (B) Shell and Tube Device

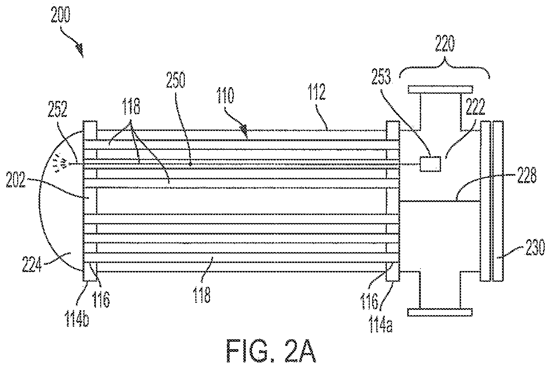

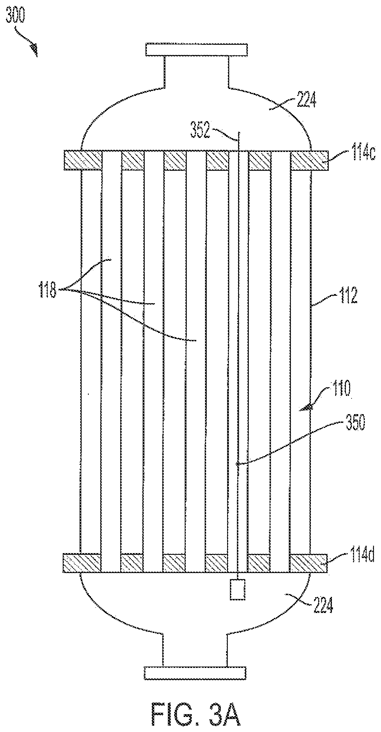

The shell and tube device 110 is shown schematically in FIG. 1. Shell and tube device 110 may form part of a heat exchanger, such as shown in FIGs. 2A and 3A. Turning now to the shell and tube heat exchangers 200 and 300 of FIGs. 2A and 3A, heat exchangers 200 and 300 generally include shell 112 defining a hollow interior, and tubes 118 positioned within the hollow interior.

By way of background, a shell and tube heat exchanger is a common type of heat exchanger used in industry. It is named for its two major components, i.e., one or more heat transfer tubes 118 mounted inside of a cylindrical shell 112. The purpose of a shell-and-tube heat exchanger is to transfer heat between two fluids. Each fluid may be a liquid or a gas. In industrial practice, it is common for at least one of these fluids to be either liquid water or steam.

Within a shell and tube heat exchanger 200, 300, one fluid flows through the interior of the tubes 118 (designated the "tube side fluid") and the other fluid flows around the outside of the tubes 118 but within the shell 112 (designated the "shell side fluid"). The heat exchanger is constructed such that the two fluids do not come into direct contact with each other. Heat is transferred from one fluid to the other by passing heat through the walls of tube 118, flowing either from tube side to shell side or vice versa. In order to transfer heat efficiently, hundreds or even thousands of tubes 118 (collectively, the "tube bundle") may be used in a single exchanger.

Shell-and-tube heat exchangers 200 and 300 also include one or more tube sheets, heads, and, optionally, other components such as baffles, tie rods, spacers and expansion joints. More particularly, tube sheets 114a, 114b, 114c and/or 114d (referred to either collectively or individually as tube sheet(s) 114) are mounted to the ends of shell 112. Tube sheets 114 are plates or forgings having planar opposing surfaces and comprising holes 116 through which the tubes 118 are inserted. The required thickness of the tube sheet 114 is primarily a function of the operating pressure of the specific shell-and-tube exchanger. The ends of the tubes 118 are secured to the tube sheet 114 by welding, or by mechanical or hydraulic expansion, such that fluid on the shell side is prevented from mixing with fluid on the tube side.

The geometry of the tubes 118 determines the number of tube sheets 114 which are required. If straight tubes are used, such as in FIGs. 1, 2A and 3A, two tube sheets 114 may be required. Alternatively, if the tubes 118 are bent into the shape of the letter "U" (known as U-tubes), only one tube sheet 114 may be required.

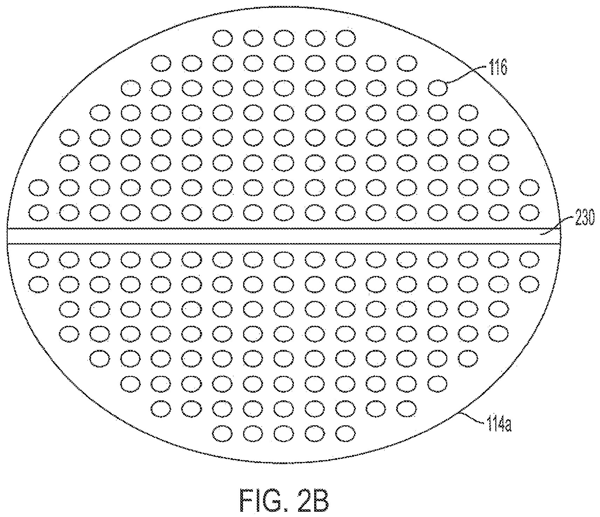

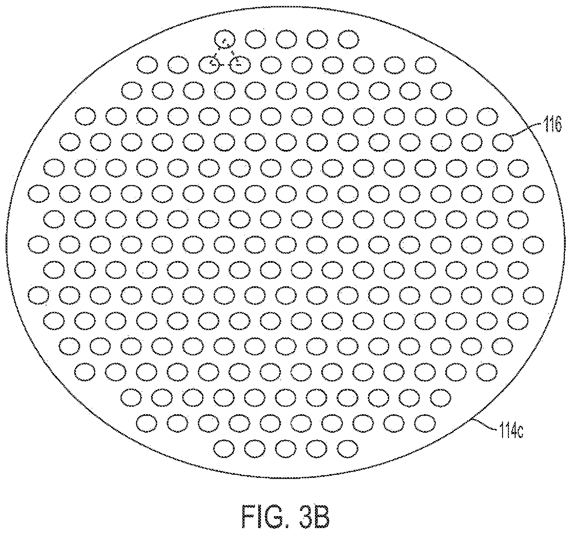

Holes 116 in the tube sheet 114 are typically arranged in one of two geometric configurations, namely, triangular or square. Tube sheets 114 utilize a fixed center-to-center distance between adjacent tubes 118 referred to as the "tube pitch." Such uniformity of the configuration simplifies exchanger design and construction. A common tube pitch is 1.25 times the outside diameter of the tubes 118. Triangular configurations (see FIG. 3B) are often used to obtain high heat transfer and compactness, whereas square configurations (see FIG. 2B) are generally preferred for services where it is necessary to regularly extract the tube bundle from the shell and clean the outside surface of the tubes.

Heads 220 are required for shell-and-tube heat exchangers to contain the tube side fluid and to provide the desired flow path through the exchanger. Typically, for each tube sheet 114 there is a corresponding head. Heads having a generally cylindrical shape are referred to as "channels" 222 (see FIG. 2A), and those having a generally domed shape are referred to as "bonnets" 224 (See FIG. 2A and 3A). In some cases, the head may also incorporate one or more pass partition plates 228 (FIG. 2A) to direct tube-side fluid flow through specific tubes. In these cases, the surface of the tube sheet 114a may further comprise grooves 230 (FIG. 2B) to stabilize the partition plates 228 and any associated sealing gaskets. Heads 220 may be welded in-place or attached to the shell 112 with flanges. Flanged bonnets or channels with removable covers 230 (FIG. 2A) are preferred in cases where it is necessary to provide access to tube sheet 114 and tubes 118 for maintenance and inspection.

Shell and tube heat exchangers 200, 300 are used broadly throughout industry, finding use in electrical power generation, industrial refrigeration, and petrochemical processing, to name a few. Shell and tube heat exchangers may be installed in a horizontal orientation (FIG. 2A) or a vertical orientation (FIG. 3A). By convention, within industrial facilities, shell-and-tube heat exchangers are named on the basis of their process function. For example, typical industrial applications of shell-and-tube heat exchangers include a condenser, reboiler, preheater, boiler, superheater, quench exchanger, Transfer Line Exchanger (TLE), evaporator, waste heat boiler, recuperator, cross-exchanger and process heater. Often, multiple heat exchangers are used within a single industrial system; for example, industrial refrigeration systems may comprise both evaporators and condensers, and petrochemical distillation systems may comprise both reboilers and condensers.

Further information regarding shell-and-tube heat exchangers may be found in Perry's Chemical Engineers' Handbook, 6th Ed., 2008, especially Section 11: Heat-Transfer Equipment and associated Figures 11-1 and 11-2.

### (C) Alternative Applications for Shell and Tube Device

The shell and tube device 110 may also be incorporated into other industrial apparatus / process systems, such as those described hereinafter.

High strength shell and tube heat exchangers, comprising U-tube bundles, may be employed as steam generators for nuclear power plants, such as disclosed in U.S. Patent No. 4,200,061.

The shell and tube device may be incorporated into a falling film exchanger, such as the falling film melt crystallizers used to purify (meth)acrylic acid.

The shell and tube device may be incorporated into a reaction system as a closely-coupled quench exchanger that is used to rapidly cool temperature-sensitive products such as Hydrogen Cyanide or Nitrogen Oxides as they exit the reaction zone, such as disclosed in US Patent No. 6,960,333. Similarly, Transfer Line Exchangers (TLE's) are used to rapidly cool high-temperature process gas as it exits an ethylene furnace.

Within the chemical manufacturing industry, the shell-and-tube device 110 may also be utilized as a chemical reactor. Within these so-called "shell-and-tube reactors" (also known as "fixed-bed reactors"), the tube side fluid typically comprises chemical reactants which are converted into one or more chemical products. Generally, commercial scale shell-and-tube reactors are large pieces of equipment comprising from 1,000 to 50,000 tubes and having tube sheets that range from between 1 to 10 meters in diameter. At such a scale, the heads of these shell-and-tube reactors can easily enclose a volume large enough for workers to physically enter and perform work and, when the shell-and-tube reactor is vertically oriented (as shown in FIG. 3A), the upper planar surface of the top tube sheet 114c may become a de facto "floor" for the enclosed work area.

Frequently, one or more particulate catalysts are placed inside the tubes of a shell-and-tube reactor to promote formation of the desired chemical products. By passing a heat transfer fluid through the shell side of the shell-and-tube reactor, the tube-side reaction temperature may be tightly controlled to maximize product yield and extend catalyst life. Unique tube configurations and shell-side baffle designs may also be utilized to further optimize temperature control.

The chemical conversions performed within shell-and-tube reactors may be exothermic (heat releasing) or endothermic (heat absorbing) reactions. In the case of highly exothermic reactions, such as for example hydrocarbon oxidation reactions, it is common for high-boiling-point fluids such as molten inorganic salts, kerosene, or organic heat transfer fluids (e.g., DOWTHERM ^{™}) to be used as the shell side fluid. Custom mechanical design features and specialized materials of construction for tubes and tube sheets are also typically used to ensure safe operation at elevated operating temperatures and pressures used for the chemical reaction.

The production of acrylic acid is but one well-known example of a commercial hydrocarbon oxidation process employing shell-and-tube devices as reactors. The chemical conversion involves two sequential, exothermic reaction steps in which propylene is first oxidized to the intermediate acrolein and then the acrolein is further oxidized to acrylic acid. Numerous solid Mixed Metal Oxide (MMO) particulate-type catalysts have been developed to facilitate this two-stage oxidation process and methods for preparing these catalysts are well documented in the literature. Fixed catalyst beds are assembled in the reactors by loading one or more particulate-type catalysts into the tubes of the reactor. As the process gases flow through the tubes, the gases come into direct contact with the MMO catalyst particles and the heat of reaction is transferred through to tube walls to the shell-side coolant.

At the present time, commercial-scale propylene-to-acrylic acid processes use one of three primary configurations of shell-and-tube type reactors: Tandem reactors, Single Reactor Shell ("SRS") reactors, and Single Shell Open Interstage ("SSOI") reactors. As a group, these commercial shell-and-tube reactors may comprise from about 12,000 up to about 22,000 tubes in a single reaction vessel, and may have production capacities of up to 100 kT/year (220,000,000 pounds per year) of acrylic acid. Certain large-scale commercial reactors may comprise from 25,000 up to about 50,000 tubes in a single reaction vessel, with production capacities of up to 250 kT/year (550,000,000 pounds per year). US Patent No. 9,440,903, provides descriptions of each of these three reactor configurations and their respective capabilities for producing acrolein and acrylic acid.

The production of ethylene oxide is another example of a commercial process employing a shell-and-tube device as a reactor. The shell and tube device 110 may be provided in the form of a commercial ethylene epoxidation reactor, comprising for example up to 12,000 tubes. These tubes are typically loaded with epoxidation catalysts comprising silver and additionally a promoter component, such as rhenium, tungsten, molybdenum and chromium, and a coolant is circulated through the shell side of the reactor. Reference is made to U.S. Pat. No. 4,921,681 and U.S. Pat. App. Nos. 2009/0234144 and 2014/0135513.

The oxychlorination of ethylene to 1,2-Dichloroethane (also known as EDC) is yet another example of a chemical process employing shell-and-tube devices. In this process, the tubes within the shell and tube device 110 are typically loaded with particulate catalysts comprising cupric chloride (so-called "Deacon" catalysts) and a coolant is circulated through the shell side of the reactor. In some embodiments, the oxychlorination reaction system may comprise two or more shell and tube devices in series. Reference is made to U.S. Pat. No. 6,180,841, U.S. Pat. No. 3,892,816, and U.S. Pat. No. 5,905,177.

Many other commercially important gas-phase catalytic reactions are performed in shell-and-tube reactors including: the conversion of propylene to acrolein and/or acrylic acid (as described above); the conversion of propane to acrolein and/or acrylic acid; the conversion of glycerol to acrolein and/or acrylic acid; the conversion of tert-butanol, isobutene, isobutane, isobutyraldehyde, isobutyric acid, or methyl tert-butyl ether to methacrolein and/or methacrylic acid; the conversion of acrolein to acrylic acid; the conversion of methacrolein to methacrylic acid; the conversion of o-xylene or naphthalene to phthalic anhydride; the conversion of butadiene or n-butane to maleic anhydride; the conversion of indanes to anthraquinone; the conversion of ethylene to ethylene oxide (as described above); the conversion of propylene to propylene oxide; the conversion of isobutene and/or methacrolein to methacrylonitrile; and the oxychlorination of ethylene to 1,2-dichloroethane.

Thus, it should be evident to one of ordinary skill that the method of the present invention is envisioned to find application in any shell-and-tube device, including but not limited to a chemical reactor, preheater, boiler, superheater, reboiler, condenser, evaporator, recuperator, Quench Exchanger, Transfer Line Exchanger (TLE), cross-exchanger, waste heat boiler, steam generator, falling film exchanger and process heater.

### (D) Shell and Tube Maintenance Background

Because of the large number of tubes 118 in a shell and tube device, it takes significant time to complete maintenance and inspection work for each shell and tube device. It is also arduous to track the status and progress of the maintenance task. Omission errors and performance errors can be substantial problems.

At the outset, it is noted that the term "omission error" as used herein means the failure to perform a specific maintenance task on a tube 118. For example, an operator could unintentionally skip a tube, resulting in a tube that may not be cleaned, inspected, or loaded with catalyst. The probability of omission errors increases with the number of tubes within the shell-and-tube device and with the duration of the maintenance activity. Many process owners generally believe that omission errors can only be prevented through steps such as a) continuous monitoring/supervision of the labor performing the activity, or b) 100% inspection after the activity is 'complete'. The inventive method described herein functionally provides continuous monitoring/supervision of the labor performing the activity, minimizing the need for 100% inspection.

In contrast, a "performance error" refers to performing a task with insufficient quality, or only partially-completing that task. Examples of performance errors include taking tube-wall thickness measurements with an improperly calibrated probe; removing rust from only the first 15 feet of a 20-foot-long tube; or filling tubes with the incorrect type of catalyst. Performance errors tend to be relatively insensitive to the number of tubes within the shell-and-tube device. Additionally, performance errors often affect large numbers of tubes at one time. For example, filing all tubes with material sourced from the same, incorrect pallet of catalyst drums. Addressing omission errors with the method of the present invention both improves efficiency and also makes available more supervisory resources for the prevention of performance errors.

There are many maintenance activities that may be performed on the tubes of shell and tube devices. Maintenance activities may include one or more multi-step tasks, and these tasks are typically repeated for each and every tube in the shell and tube device. Examples of maintenance activities which may be beneficially monitored using the method of the present invention, include but are not limited to:
a) Inspection Activities: video inspection for cleanliness and/or mechanical damage; thickness Measurement (e.g., Eddy-current inspection); identification of Blocked Tubes (e.g., IR detection of low-flow tubes; and identification of Organic contamination via reflected UV light inspection.
b) Cleaning Activities: Sand blasting, CO2 Pellet Blasting, Hydroblasting, Liquid Nitrogen Blasting, Drilling, Wire Brushing, Pigging, and Removal of Coke Accumulations.
c) Repair Activities: Re-welding tube-to-tubesheet welds; and Mechanical Plugging of tubes.

For shell and tube devices used as reactors, maintenance activities may also include those activities associated with catalyst changes. Examples of catalyst change activities which may be beneficially monitored using the method of the present invention, include but are not limited to:
a) removal of catalyst from tubes, for example using an "air lance," a fish tape, or a vacuum hose,
b) visually verifying that tubes are empty (i.e., "Light Check"),
c) installation of heat transfer inserts into tubes,
d) installation of catalyst retainers, for example Catalyst Springs or Catalyst clips,
e) loading of ceramic or metallic inert particles into tubes,
f) loading of one or more layers of catalyst into tubes,
g) measurement of catalyst bed outages, and
h) measurement of catalyst load pressure drop (dP).

### (E) Imaging System Details

Further details of imaging system 120 of the system 100 are described hereinafter.

### (a) Number and Arrangement of Imaging Devices

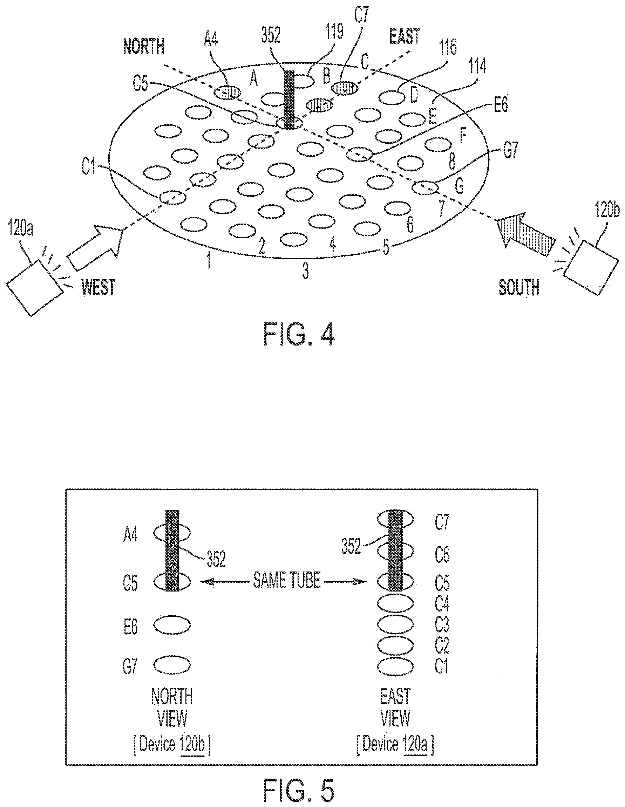

**The system 100 preferably comprises two imaging devices 120a and 120b, as they are arranged in** **FIG. 4****, for example, i.e., above the plane of tubesheet 114 and located in an east-west direction and a north-south direction, respectively.**

**Alternatively, imaging system 120 can comprise a single imaging device 120a that is located above the tubesheet 114. For example, a single imaging device 120a can be arranged in a** catadioptric system that is employed to achieve two views of the same object (i.e., tubesheet 114) with a single imaging device. This essentially constitutes single camera stereo-vision, which is accomplished using prisms and/or mirrors, e.g., planar, parabolic, or spherical mirrors. In addition to using fewer physical cameras, any issues with synchronizing the collection of images are eliminated. In the simplest embodiment, a right-angle mirror is placed directly in front of the **imaging device 120a** to act as a beam splitter, thereby creating a right hand field of view and a left hand field of view; and then additional mirrors are positioned to allow the right side and left side views to acquire an image of the same object from two different viewing angles. A catadioptric system is disclosed in U.S. Patent App. Pub. No. 2011/0143287, which is incorporated by reference in its entirety. A catadioptric system is also disclosed in MULTI-MIRROR SYSTEM FOR HIGH-SPEED CAMERA MONITORING APPLICATIONS, PROBLEMY EKSPLOATACJI - MAINTENANCE PROBLEMS, Garbacz, 2013, which is incorporated by reference in its entirety. See, e.g., figure 3 of that article. A catadioptric system is also disclosed in MONOCULAR STEREO MEASUREMENT USING HIGH-SPEED CATADIOPTRIC TRACKING, Sensors, Shaopeng Hu et al., 2017, which is incorporated by reference in its entirety.

### (b) Imaging Device types

**Each imaging device may be a detector, such as a photodetector or a thermal detector. A photodetector further comprises a plurality of light sensors, known as picture elements or "pixels". Similarly, a thermal detector comprises a plurality of heat sensors, known as microbolometers or simply, bolometers.**

The most common and preferred embodiment incorporates optical imaging. In the optical imaging embodiment, an imaging device comprising a photodetector and an image processing software package are used for imaging with visible light. Imaging devices 120a and 120b may each be a digital camera, an RGB color video camera, or a black and white camera, for example. Optics, i.e., lenses, focus light on a photodetector located within the focal plane of the camera (this is the so-called Focal Plane Array or FPA) to obtain images with minimal distortion (i.e., in-focus images). The individual sensors within the photodetector, (i.e., pixels), convert light contacting the photodetector into a digital signal. The digital signals are then transmitted to the image processor, wherein a digital image of the combined digital signal data is represented as a mathematical array.

When a digital image of the tube sheet 114 is acquired, it may comprise many thousands, or even millions, of digital values, depending on the detector array used. For example, a typical "4K" color digital Camera will comprise a CMOS photodetector array having 3840 horizontal pixels by 2160 vertical pixels, resulting in 8,294,400 distinct color measurements; this is generally referred to in the art as an "eight-megapixel array" or simply an "8MP" detector.

As known in the art of digital imaging, optics and detector size control how much of the physical world can be "seen" by the imaging device, a term known as the Field Of View (FOV). Detectors are commonly configured as a fixed array (grid) of individual detection elements, with larger numbers of detection elements supporting a wider field of view and/or greater resolution. Most commercial photodetectors are implemented as a flat array built upon silicon wafers, which means that the maximum physical size of available silicon wafers limits the total number of detection elements possible; once the maximum array size is reached, only the selection of the lens(es) can impact imaging device resolution and the width of the field of view (FOV).

By convention, camera lenses are typically described by their horizontal FOV angle and their vertical FOV angle, while photodetectors are typically described by the number of pixels in the horizontal and vertical dimensions of the detector array. Because there are a fixed number of picture elements (pixels) in a given photodetector, the FOV and image resolution are inversely related, i.e., a wider FOV (more image area seen by the detector) results in a lower resolution, whereas a narrower FOV (more pixels per unit of image area) results in a higher resolution. Selection of an appropriate detector size (i.e., total number of pixels) and an appropriate lens FOV is within the ability of one of ordinary skill in the art of digital imaging.

Still-image digital cameras may be used to acquire optical images, but video cameras are often easier to configure for use with a networked computer. Commercially available video cameras are typically constructed with the built-in capability to transfer image data to an image processor (e.g., laptop computer) via Wi-Fi, LAN / PoE (Power over Ethernet) wiring, fiber optics, etc. In some embodiments, at least a portion of the image processing may be performed within the circuitry of the camera to speed up processing/reduce the amount of data to be transmitted (and hence lower the bandwidth requirement).

### (c) Energy Transmission

The imaging system 120 can detect visible light energy, however, the general concepts described above apply to all forms of energy transmission (e.g., light, heat, pressure, sound, x-rays, radio waves, electron beams) and their appropriate purpose-specific detectors.

If the energy is light reflected off the surface of the object (e.g., wavelengths of light selected from one or more of the visible light spectrum, the infrared spectrum, or the ultraviolet (UV) spectrum), a photodetector array (e.g., a Silicon-based CMOS photodetector, comprising an array of individual sensors known as pixels) can be used to measure the intensity of light at said one or more wavelengths and to create a monochromatic (grayscale color) digital image or an "RGB" color digital image. Using appropriate Data Visualization software (e.g., software known as display drivers), the color data may be optionally rendered as a visual image on a display device.

The source of the reflected light may be from the environment (e.g., sunlight) - known as passive illumination - or the light may emanate from an artificial white light source (e.g., a lamp) - known as active illumination. The light source may emit wavelengths of light within one or more of a visible light spectrum, an infrared (IR) spectrum, or an ultraviolet (UV) spectrum.

If the energy is thermal energy emitted from the object (e.g., IR radiation at wavelengths of between 7.5-14 µm), a thermal imaging system 120, comprising sensors known as bolometers, can be used to create a digital image comprising temperature values. Using appropriate Data Visualization software, the temperature data may be optionally rendered as a thermographic (visual) image on display device 140. Note that the infrared energy is emitted/radiated from the object, so there is no illumination source per se.

If the energy is reflected radio waves (e.g., from a radar system), the resulting digital image comprises radio signal return-time values that represent the distance between a point on the object and the radio-wave detector (receiver). When used with the inventive method, radar operating in the EHF band (also known as millimeter-wave radar) is preferred. Image acquisition systems based upon Radar, Sonar, Lidar, and the like are known herein as Non-contact Ranging Devices (NRD's), which generally "paint" the surface of an object with a moving energy beam in order to collect a large number of closely-spaced return-time (distance) measurements. Using Data Visualization software, this distance data can optionally be rendered as a visual image on display device 140 (e.g., weather-radar displays or LIDAR topographical maps). By their nature, NRD's require active "illumination" with energy that can then be reflected back.

### (F) Software Details

Software code to perform the image-processing steps described herein may be written using a variety of computer programming languages, for example, using C++, Python, or MATLAB programming languages. The image-processing steps employed may include one or more techniques widely known in the art of digital image processing, such as filtering, conversion of pixels between color and grayscale, (Canny algorithm) edge detection, Circle Hough Transforms, conversion of image data from one color model to another (e.g., RGB to L*a*b*), creation of image masks, and color detection. Libraries of standardized functions to efficiently perform these image-processing steps have been created and are currently available for incorporation into programming code, greatly simplifying the preparation of software routines. OpenCV *(Open Source Computer Vision Library:* http://opencv.org) is one such library of image-processing functions, which at present is available for download as open-source software. Although initially written under the C++ programming language, so-called "wrappers" are now available to allow functions in OpenCV to be used with other programming languages, such as Python, JAVA, and MATLAB. Proprietary applications such as IMAGE PROCESSING TOOLBOX^{™} and COMPUTER VISION TOOLBOX^{™} (commercially available from The MathWorks, Inc of Natick, Massachusetts, USA) may be used to implement image-processing described
herein. OpenCV adapted for use with the Python language (also known as OpenCV-Python) may also be used for image processing. Enhancements to Python, known as the "Numerical Python extensions" or "NumPy", may also be utilized to improve the performance of mathematical operations with array data.

Image processing software, such as Matlab and OpenCV, can perform operations using many different color models. As is known in the art, "Color models" are abstract mathematical representations of colors using ordered lists of parameters, referred to herein as "Channels." Images can be represented in many different formats, corresponding with well-known color models including RGB, HSV, and L*a*b*. Colors represented in the RGB color model specify the intensity of each of the three channels: R (Red), G (Green) and B (Blue) using values ranging from 0 to 255. RGB is the native format for devices such as video cameras and televisions. Colors represented in the HSV color model specify the following three channels: Hue, representing the dominant wavelength; Saturation, representing shades of color; and Value: representing Intensity. Colors represented in the L*a*b* color model specify the following three channels: L*, representing perceptual lightness or Luminosity; a*, representing the colors on an axis ranging between red and green; and b* representing the colors on an axis ranging between yellow and blue.

In contrast to full color images, Grayscale images contain only a single channel representing shades of gray. Pixel intensities in this color space are represented by values ranging from 0 to 255, with black being the weakest intensity (value of 0) and white being the strongest intensity (value of 255). Thus, the maximum number of states that can be represented by a single pixel in grayscale is 256. With only a single channel, image processing in Grayscale, rather than in full color, can be much faster and require fewer computing resources.

Image processing software further includes color-conversion algorithms, such that images acquired under one color model (e.g., an RGB image from a video camera) can be converted to a different color model. Such conversions are typically performed to simplify processing calculations or to highlight certain features within a Region Of Interest (ROI). Additionally, conversion algorithms allow color digital images to be converted to grayscale; which is often advantageous when searching for areas of high-contrast that typically occur along the edge of objects, and which is a key aspect of object-detection algorithms.

### (G) Projection-type Maintenance Activity Examples

The system 100 described herein is particularly effective in monitoring "projection-type maintenance activities" in which objects project from the tube ends 119 of shell-and-tube devices during the activity. Various "projection-type maintenance activities" are described hereinafter and represent an important subset of the maintenance activities that may be performed upon shell-and-tube devices.

In the example shown in FIG. 2A, when hydroblasting a heat exchanger, cover 230 and bonnet 224 are first removed and then a hydroblasting lance 250 is inserted into the entrance end of a tube; pressurized water is sprayed out the end 252 of the lance 250, dislodging material from the tubes 118 for cleaning purposes. Operators control the spray using a handle 253 that is connected to a supply of pressurized water. The water, and eventually the lance 250, project out the discharge end of the tube 118 at the discharge tube sheet 114b, as shown. As a safety precaution, workers are only present at the entrance end of the shell and tube heat exchanger 200, and no workers are present proximate to the discharge tube sheet 114b.

In the example shown in FIG. 3A, a fishtape 350 is inserted through the lower tubesheet 114d into the bottom of the tube 118 and the free end 352 of the fishtape 350 ultimately projects out the top (discharge end) of the tube 118 at the upper tubesheet 114c. It is noted that the operators (workers) are only in the space below the lower tubesheet 114d, and do not directly observe the fishtape 350 projecting out the discharge end of the tube 118, so they may not be certain that the end 352 of the fishtape 350 has passed completely through the tube 118. In accordance with the method of the present invention, the system 100 may be beneficially used by the workers to receive feedback to confirm the successful projection of the end 352 of the fishtape 350 out the discharge end of the tube 118 at the upper topsheet 114c.

In yet another example, foam cylinders ("swabs") are "shot" through the tubes 118 using a burst of pressurized air. This activity may be used to remove dust or moisture (e.g., liquid water) from tubes 118 following a cleaning step, such as sand blasting or hydroblasting. In such a projection-type maintenance activity, it is essential that every swab is ejected from every tube 118. Any swabs that remain in the tube could impede later activities, such as for example, catalyst loading.

Some examples of projecting objects that may be tracked during projection-type catalyst removal maintenance activities include (i) a fishtape projecting out of the top of a tube, (ii) a catalyst-removal air lance projecting out of the bottom of a tube, and (iii) a "polytube" vacuum hose projecting out of either end of a tube.

Some examples of projecting objects that may be tracked during projection-type tube inspection maintenance activities include a boroscope projecting out either end of a tube, and an eddy current probe projecting out either end of a tube.

Some examples of projecting objects that may be tracked during projection type tube cleaning maintenance activities include:
a. foam pigs, tube cleaners (e.g. from Conco Services LLC of LaPorte, Texas USA), rubber balls or other projectiles ejected ("shot") out of either tube end;
b. cleaning Media (water, etc.) from hydroblasting; sand blasting; CO2, walnut shell, or N2 blasting - ejected ("spraying") from either tube end;
c. rotary wire tube brushes projecting out of either tube end;
d. drill bits projecting out of either tube end; and
e. lances for hydroblasting; sand blasting; co2, walnut shell, or N2 blasting - projecting out either tube end.

It is generally noted that operators performing the projection-type maintenance activities described above may be located on the "entrance end" of a shell and tube device while monitoring under the present method is performed at the "discharge end" of the shell and tube device (e.g., heat exchanger or reactor). This means that, generally, there are no obscuring objects or people proximate to a tube sheet 114 that is being monitored while the projection-type maintenance activities are being performed, so it is not necessary to address obstructed views.

### (H) Process for Monitoring a Projection-Type Maintenance Activity for a Shell and Tube Device

In general, and according to one exemplary method, for a shell and tube device comprising a tube sheet including a plurality of tube ends arranged in a fixed pattern of rows and columns, a method for monitoring the status of the shell and tube device during a projection-type maintenance activity comprises the following general steps:
a) assigning a unique identifier to each of the tube ends,
b) at an initial acquisition time (Ti), acquiring one pair of initial digital images (Dai and Dbi) of at least a portion of the tube sheet from two different vantage points,
c) determining an initial state of an attribute (Ai) having at least two states for each of the tube ends within said pair of initial digital images, and
d) creating an initial data record in a relational database for each tube end within said pair of initial digital images, said initial data record including:
   i. the initial acquisition time (Ti),
   ii. the unique identifier for the tube end, and
   iii. an initial state of the image attribute (Ai) at the initial acquisition time (Ti).

Finer details of the above method will be described hereinafter.

### (a) Initial Mapping Process

Turning now to FIGs. 1, 4 and 6, and, prior to starting a projection-type maintenance activity (e.g., fishtaping), the imaging system 120 first collects (i.e., acquires) an initial image of the top plane of the tube sheet 114 to map the location of the tube ends 119. This step may be referred to as the initial mapping step (i.e., step a) above).

Imaging system 120 may for example be an optical system comprising visible light spectrum photodetectors. In collecting the images, imaging system 120 receives light through its aperture, which represents the condition of tube sheet 114, and converts the light into a set of digital measurements. As background, the acquired measurement data, formatted as an array, is known herein as a Digital Image.

Once acquired, the digital image of the tube sheet 114 is then forwarded to processor 126 of computer 124 via Wi-Fi, LAN / PoE (Power over Ethernet) wiring, fiber optics, etc. The software 132 of the computer 124 creates a unique tube identifier for each tube end 119 visible within the digital image. First, the image processing software locates the geometric center of each tube end 119. The unique identifier is then assigned to each center's (x,y) position in the image array. Preferably, each tube's unique identifier is provided as a set of Cartesian coordinates of the form (row, column), corresponding to the row and column designations used in the fabrication drawings for the tube sheet. In this way, the software 132 knows which tube(s) 118 it is viewing in the image array and can uniquely identify each one of them.

The image processing software may locate the geometric center of the tube end 119 by performing the following steps:
i. Identify all of the geometric regions of interest (i.e., the circular tube ends) within the image array using the Circle Hough Transform (CHT) function and/or Canny edge detection algorithms within OpenCV or Matlab software. It is noted that there are specific commands within the software to utilize these functions. The commands return variables representing the circle center coordinates (x, y) in the array and also the radius of the circle.
ii. Align image array coordinates with known dimensional data for the tube sheet, in order to map identified circular tube ends to the tube sheet drawing. It is noted that this step may be made easier by utilizing benchmarks in the image to orient the tube sheet drawings.
iii. Correlate the unique tube identifier (row, column) for each tube in the drawing with the (x,y) location coordinates of each circle-center in the image array.

Because the tube sheet 114 is a stationary component, it generally does not move relative to the imaging system 120. Consequently, the location of each circle center in the array does not change and this mapping step should only need to be performed once during the maintenance activity.

It should be noted that only about 1/3 of the tubesheet area of a typical shell-and-tube device actually comprises holes (tube ends), while the remaining approximately 2/3 of the tubesheet area comprises only the planar surface between the tube ends. Thus, only about 1/3 of the imaging device data represents measurements from within a so-called Region Of Interest (ROI) on the tubesheet 114. By knowing the locations of all of the tube ends within the image, subsequent processing may be limited to just these circular ROI's, significantly reducing the time to evaluate each digital image. Those of ordinary skill in the art of image processing will recognize that image "masks" may be created using image processing software and then beneficially applied to achieve such optimized image processing.

In some embodiments, this initial mapping step might be performed manually by acquiring a visible light reference-image and rendering it on a computer using "Image Viewer" software, (commercially available from The Mathworks Inc., Natick, MA 01760 - USA). A key feature of Image Viewer is its ability to display user-selected individual pixel location-values and their associated color/intensity values. This allows for manual identification of the specific pixels that fall within each tube end, thereby providing a method for correlating groups of pixels with the appropriate unique tube identifier. This approach is most beneficial when the shell-and-tube device comprises a relatively small number of tubes.

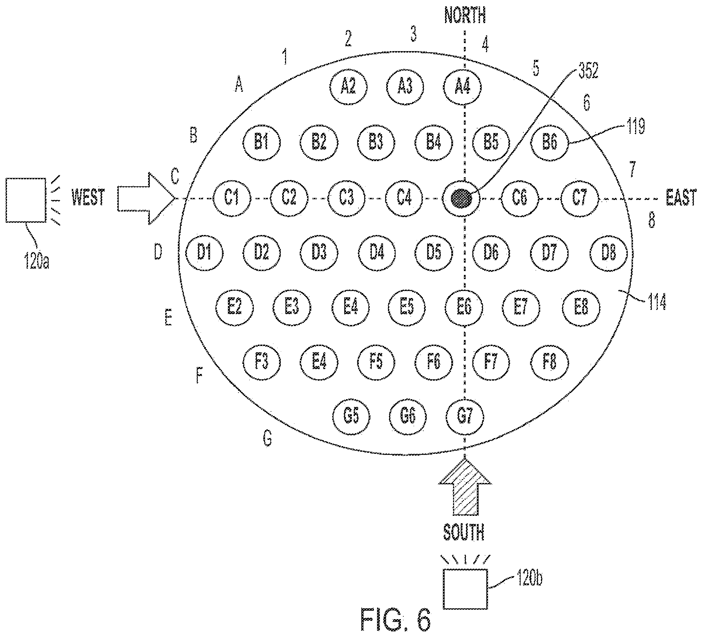

It is generally noted that either one or both devices 120a and 120b may be employed in the system 100, however, for purposes of this description, it is assumed that both devices 120a and 120b are utilized in the system 100. As shown in FIGs. 4 and 6, one imaging device 120a acquires an image from west to east, and the other imaging device 120b acquires an image from south to north. Accordingly, both devices 120a and 120b cover all four quadrants of the tube sheet 114.

It is also noted that tube sheet 114 may or may not be actively illuminated, and the devices 120a and 120b may or may not be first aligned with particular rows and columns of tube ends 119. Lastly, the devices 120a and 120b may be digital cameras with visible light, IR and/or UV light spectrum detectors. Due to differences in emissivity, thermal IR cameras may in some cases be used.

### (b) Monitoring Process

Prior to commencing the fishtaping maintenance activity (for example), devices 120a and 120b concurrently acquire initial pairs of images (Dai and Dbi) of the tube sheet 114. This constitutes step b) of the above method. The computer 124 then determines an initial state of an attribute (Ai) for each of the tube ends 119. This constitutes step c) of the above method. By way of background, an attribute describes an identifiable quality (shape, color, etc.) that is capable of being monitored. It is noted that any attribute can be measured by the selected imaging devices, such as:
a) either a change, or an apparent change, in the shape of the circular tube end 119, for example, due to a generally-rectangular fishtape appearing to be on top of the circlular tube end, i.e., the detected shape changes from an "O" shape to a shape resembling the Greek Letter "Φ" (Phi),
b) a color, luminosity or darkness difference between the tubesheet 114 and the object projecting out of the tube 118, and
c) the appearance of a new object using, for example, canny edge detection. It is noted that edge detection will identify the outline of a new object, such as the border of the generally-rectangular fishtape 352.

Thus the attribute being monitored may be shape, color, or the presence of an object. There may also be many other useful attributes that could be assessed.

At this early stage of the process, the initial state of the attribute (Ai) should signify that no objects are projecting from the tubes 118 because the fishtaping process has not yet begun. The state may be expressed numerically. Simply stated, this step establishes an initial baseline of the state of the tubes 118.

At step d) of the above method, the computer 124 creates an initial data record in relational database 134 for each tube end 119 within the initial pair of digital images. The initial data record includes the initial acquisition time (Ti), the unique identifier for the tube end, and the initial state of the selected image attribute (Ai) at the initial acquisition time (Ti).

After steps a) - d) are complete, system 100 monitors for changes at the tube ends 119 by continuously acquiring concurrent pairs of digital images of the tube sheet 114 using the devices 120a and 120b. For many projection-type maintenance activities, changes can be quite rapid. It is therefore preferred that the pairs of images be acquired at a high frequency, such as for example 1 pair of images per second, or 60 pairs of images per second, or even 180 pairs of images per second. In one embodiment, two Aida model # UHD-100A RGB digital cameras (commercially available from AIDA Imaging, Inc of West Covina, CA. 91797 - USA (www.Aidaimaging.com)), are used to acquire pairs of digital images at a rate of 30 per second.

Turning to FIGs. 4-7, during a projection-type maintenance activity (e.g., fishtaping), transient changes of the attribute within the image will be caused by an object (fishtape end 352) passing through the plane of the tubesheet 114 and projecting out of the end of a tube 118. Successive images (Tx > Ti) of the tubesheet 114 are compared with each other to detect a transient change in an attribute at the Region of Interest, i.e., proximate to the circular tube ends, but not necessarily only within the x,y plane of the tubesheet 114.

In one embodiment, tube sheet images are collected at a high frequency - for example at a rate of 60 images per second - and monitored for changes between successive images indicating that motion has occurred on the tube sheet. This process may be referred to in the art as "optical motion detection." If no motion is detected, then no further image processing is performed, thereby optimizing computer-resource use.

It is noted that motion detection does not necessarily require a pair of images. Motion detection constitutes a motion "trigger" for deciding when to take further image processing action and does not need to determine exactly where the motion occurred. More particularly, if no motion is detected, then no further image processing is performed with a specific image pair. Simply stated, there is no need to assess the state of each tube, when nothing has changed. This eliminates wasted time processing images and filling up a database with unnecessary data. Conversely, if motion is detected, then all of the necessary image processing according to the exemplary method described herein is performed in order to determine the state of each tube end. It is possible, for example, that fishtapes are projecting from more than one tube at the same time and then the states are recorded (along with the unique tube identifier) in the database.

It is also noted that the "motion trigger" may be a change in color (for example) anywhere on the tubesheet, while the tube-end attribute (A) may instead be the shape of the tube ends. The two attributes can serve different purposes and need not be the same. The color of pixels in an image is faster to assess with image processing software than shapes in an image, which makes color a more responsive motion trigger. Conversely, while shape recognition may result in slower processing, it may be more accurate for identifying the specific tube from which a fishtape projects. This is because assessing the circular tube end necessarily involves using a large number of pixels within a field of view; using a group of pixels for assessment will utilize mathematical averaging, making it inherently less sensitive to noise.

FIGS. 4, 5 and 6 depict the views acquired by the pair of devices 120a and 120b during the fishtaping maintenance activity. Each image in the pair is acquired at the same time, and each image represents a different view.

In this example, at some point in time, the end 352 of the fishtape 350 protrudes out of the tube 118 at tube position 'C5' and above the plane of the tubesheet 114 (see FIG. 6). This change is captured by devices 120a and 120b. It is noted that the tubes 118 are arranged in rows A-G and columns 1-8; and, in this example, fishtape end 352 is located at row C and column 5.

Referring to FIGS. 4 and 5, device 120b detects the presence of the fishtape end 352 (e.g., an object) at tubes located at C5 and A4. The attribute that is being monitored here may be shape, for example. It is noted that device 120b detects the presence of the fishtape end 352 at the tube located at A4 (even though the fishtape end 352 is not positioned at location A4) due to the orientation of device 120b and fishtape end 352. Device 120a detects the presence of the fishtape end 352 at tubes located at C5, C6 and C7. It is similarly noted that device 120a detects the presence of the fishtape end 352 at tubes located at C6 and C7 (even though the fishtape end 352 is not positioned at locations C6 and C7) due to the orientation of device 120a and fishtape end 352.

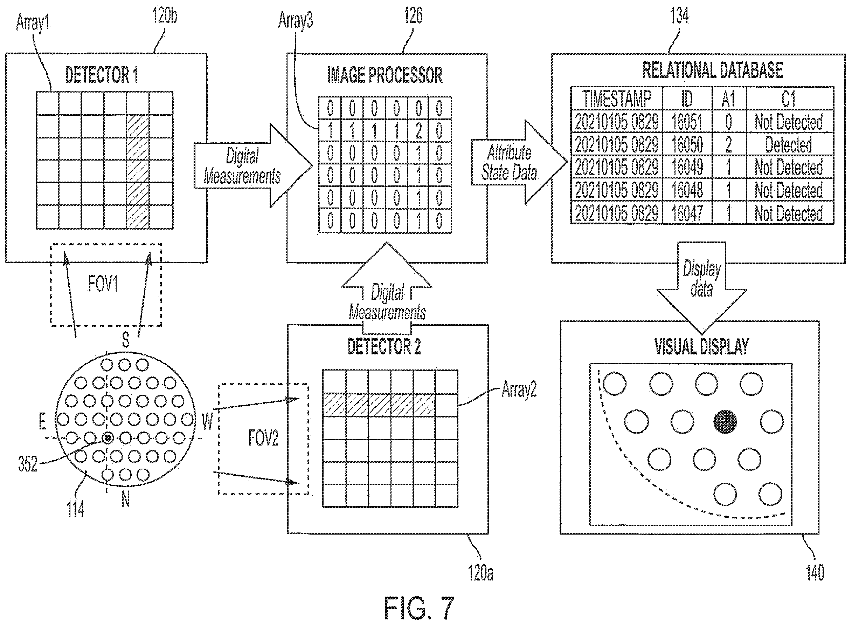

Turning back to the monitoring method, both devices 120a and 120b transmit this information as digital images (see FIG. 7) to the image processor 126 of computer 124. The digital images may be in the form of two arrays (i.e., array 1 and array 2). Computer 124 compares the data transmitted by both devices 120a and 120b and determines that both devices 120a and 120b have detected an object (i.e., fishtape end 352) at the tube 118 located at C5 (see value '2' at array 3 of image processor 126 at FIG. 7). It should be understood that the location of tube 118 at C5 (as well as all of the other tubes) was established in the initial mapping step.

The computer 124 performs image processing on each pair of images provided by devices 120a and 120b by adding array values, subtracting array values, or performing other mathematical manipulations known in the art, for example, in order to assess the state of all of the tubes 118 within the images. In the example shown in FIG. 7, the image processor 126 adds the values of corresponding cells in the two detector arrays 1 and 2 (thus, 0+1 =1, 1+1=2, etc.) to arrive at array 3. Accordingly, computer 124 concludes that a projecting fishtape end 352 is present at the tube 118 located at C5.

Computer 124 then assigns the appropriate state values (0, 1, 2) for a tube-end shape Attribute (A1) of each tube. In the example shown in FIG. 7, the value 2 indicates non-circular shapes in both images, 1 indicates a non-circular shape in only one of the images, and 0 indicates only circular shapes in both images. These state values are then transferred to the relational database 134 for storage and analysis. The relational database 134 maps attribute states (A1) to condition values (C1). For example, a state value of '2' maps to the condition "Detected"; whereas values '1' and '0' map to the condition "Not Detected." The relational database 134 software calculates performance metrics for the activity (e.g., % complete, number out-of-spec, predicted completion time, time stamp, tube identification "ID").

The performance metrics are (optionally) transferred to visual display 140 (such as a digital computer monitor or a printer) for real-time reporting. Data visualization software may also be used to render a visual representation of measurements within the digital image.

In one embodiment, the computer 124 uses the pair of images acquired by devices 120a and 120b to form a single, enhanced digital image. Typically, visual elements found within both images of the pair are maintained in the enhanced digital image, while visual elements found in only one of the two images of the pair are omitted from the enhanced digital image. The enhanced digital image may optionally comprise supplemental data from one or more additional imaging devices (not shown), from a preceding pair of images from devices 120a and 120b, or even from the initial pair of digital images (Dai and Dbi). The use of an enhanced digital image may be beneficial when processing image data with noise, distortion, or blurring, such as may for example occur when tracking fast-moving objects, such as projectiles. Optionally, the enhanced digital image may be archived within the memory devices of computer 124 for later use in improving the image processing algorithms and/or adjusting attribute assessment parameters. Once formed, the enhanced digital image may be used to assess the state of each tube within the pair of digital images, with computer 124 assigning the appropriate state values (S0, S1, S2,...) for an attribute of each tube.

Although described with respect to fishtapes projecting from reactor tubes, one of ordinary skill could easily apply the inventive method to the monitoring of many other projection-type maintenance activities, such as for example clearing polymer from reboiler tubes using a waterblasting lance, or descaling heat exchanger tubes using air pressure-driven projectiles (e.g., pigs).

### (c) Data Visualization Process

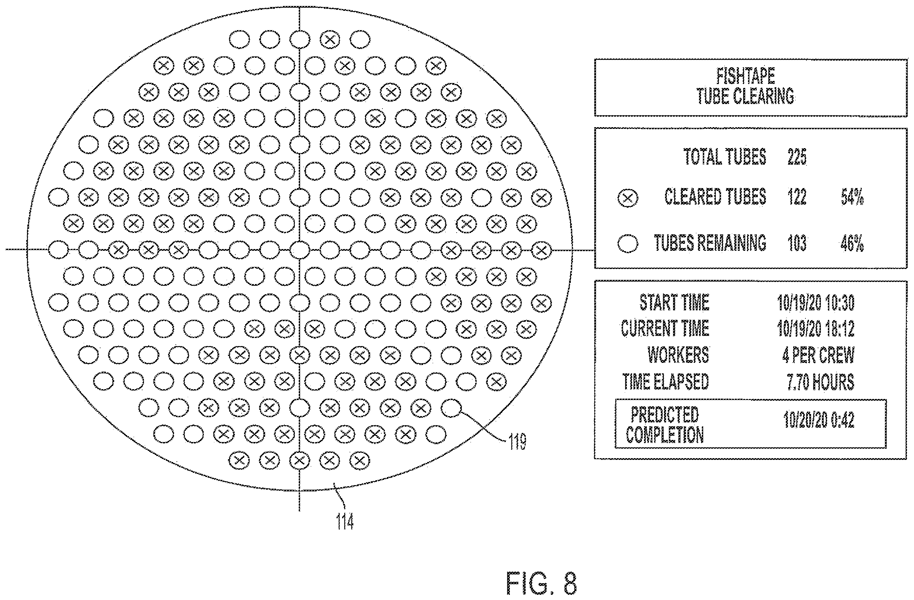

Turning now to FIG. 8, data visualization software may be used to convert the digital image data into a format appropriate for rendering as a visual image [i.e., a picture] on a display 140. In addition to visual images, data visualization software may also be used to present digital image data in one or more summary formats, such as a table, graph, spreadsheet, or color-coded summary graphic. Performance metrics may be transferred to visual display 140 (such as a digital computer monitor or a printer) for real-time reporting. Data visualization software may also be used to render a visual representation of measurements within the digital image.

Using the relational database 134 software to evaluate maintenance behavior for all tubes during a specific time period, it is possible to (i) generate performance metrics, such as "number of tubes cleared" or "percent of tubes cleared," and (ii) predict future behavior, such as the remaining time to complete the maintenance activity. Furthermore, by assessing all of the tubes in this manner, the overall condition of the tube sheet 114 at the end of the activity (e.g., 54% of tubes cleared) can be determined, and a database record of that result can be created for future reference. The computer 124 may further be configured to calculate a predicted completion time as a function of the start time, current time, total number of tubes, and total tubes cleared (or remaining).

The system 100 may also be used as a real-time display interface that is capable of communicating the state of each tube 118 at a specific time via the display 140. Visualized on the display 140 is the fishtaping maintenance performed on each tube 118 of the tube sheet 114. The display interface may include a representation of the tube sheet 114 using symbols or colors. The display interface may optionally include key performance metrics, such as cleared tubes, tubes remaining, percent of tubes inspected or the like, which are calculated using data records from the relational database 134. The display interface might also include access to related information from the relational database 134, such as the device name or a description of the task being performed.

Additionally, it may be beneficial to provide one or more portable display devices to operators within the workspace of the shell and tube device (e.g., reactor), so that they can monitor the state of tubes during job execution. For example, workers performing fish taping from a position below the lower tube sheet may benefit from the capability to monitor, in real-time, the behavior of the tube ends within the upper tube sheet. If used, it is preferred that such display devices are configured as wireless (Wifi) display devices. It is also preferred that the display devices utilize touch-screen capabilities for ease-of-use in the field.

### (I) Image Metadata and Workspace Parameters

As described above, data within a digital image is processed to determine attribute details about each tube end 119 in the image, as was described above. Generally, an attribute is a feature within the image, such as shape, color, intensity, and/or texture, for example. Each attribute can generally be described by the presence or absence of one or more specific states. Time-stamped data about each tube, including its identifier and its attribute details, are stored in relational database 134 (SQL Software or similar) for later analysis. In one embodiment, the time stamp is provided in Julian date format.

Additional image information, herein referred to as Image Metadata, may also be stored in the relational database. Image Metadata may optionally include GPS coordinates, camera number, a job description (e.g., "July 2020 inspection"), and/or the shell and tube device I.D.

Workspace parameters may also be stored in the relational database 134, as will be described hereinafter. More particularly, and as previously noted, commercial scale shell-and-tube reactors may have tube sheets that range from between 1 to 10 meters in diameter. At such a scale, the heads of these shell-and-tube reactors can easily enclose a volume large enough for one or more workers to physically enter, creating what is known in industry as a "confined workspace." During Maintenance Activities, the environment within such confined workspaces may be controlled in order to prevent damage to the catalyst, minimize the formation of rust inside the reactor, and protect workers from potential hazards. When performing Maintenance activities, it may therefore be beneficial to measure one or more workspace parameters in order to better control the confined workspace environment.

For example, climate-controlled air (heated or cooled) may be supplied to the confined workspace in order to maintain a preferred internal temperature and/or control relative humidity within the reactor. In one embodiment, one or more temperature measurement devices may be placed within the ductwork of the climate-control system and/or within the confined workspace. In another embodiment, one or more Wi-Fi enabled sensors may be temporarily placed within the confined workspace to continuously monitor the relative humidity (%RH) therein. Time-stamped temperature measurements and/or time-stamped %RH measurements may then be automatically communicated through wired or wireless means to computer 124, stored in the relational database 134, and optionally presented on the visual display 140.

In another example, portable gas analyzers may be used to continuously monitor the confined workspace atmosphere to detect the presence of harmful gases (using so-called "toxic gas detectors"), verify that sufficient oxygen concentration is maintained (using so-called "oxygen meters"), and/or monitor for flammability hazards (using so-called "LEL monitors"). Conventionally, such atmospheric monitoring activity is performed by an individual known as a "hole watch", with analyzer measurement data typically being recorded by hand on paper logsheets. However, in the preferred embodiment, time-stamped measurements from such gas analyzers may be automatically communicated through wired or wireless means to computer 124, recorded in the relational database 134, and optionally presented on visual display 140.

In accordance with safety regulations, it is typically necessary to track the number of workers within a confined workspace and to account for them in the event of an emergency evacuation. Conventionally, this activity is also performed by a "hole watch", again typically using handwritten logsheets. However, in a preferred embodiment, one or more LiDAR devices, such as for example a Density Entry Sensor (available from Density Inc. of San Francisco, CA, USA), may be mounted above entry points, such as manways in the reactor head, to automatically track personnel entering / exiting the workspace. By continuously communicating time stamped entry and exit data through wired or wireless means to computer 124, it is possible to determine in real-time the number of personnel within the workspace during maintenance activities. Storing this time-stamped workspace occupancy data in the relational database 134 allows manpower performance metrics to be calculated, including for example, manpower efficiency-factors and the duration of any work stoppages. Although the specific examples described herein illustrate the application of the present method to a chemical reactor or a heat exchanger, for example, one of ordinary skill in the art could easily envision a similar approach being applied to other shell-and-tube devices.

Additionally, for simplicity, the inventive method has been described above as using optical devices (e.g., cameras) within imaging system 120. In some embodiments, however, imaging system 120 comprises at least one Non-contact Ranging Device (NRD), such as for example a radar device, a sonar device, a laser scanning (LiDAR) device, or an electron-beam device.

For example, in a preferred embodiment, imaging system 120 comprises a Velarray M1600 solid-state LiDAR device (available from Velodyne Lidar of San Jose, CA - USA), and MATLAB software, which includes a "velodynelidar" interface, is used for image processing and optionally, for visualization of associated point clouds.

While this invention has been described with respect to at least one embodiment, the present invention can be further modified within the scope of the invention as defined by the appended claims. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

## Claims

1. A method for monitoring a status of a shell and tube device during a projection-type maintenance activity, wherein said method comprises:
- acquiring an initial digital image (Di) of a tube end on at least a portion of a tube sheet of the shell and tube device;
- acquiring a later digital image (Dx) of the tube end at a later time during the projection-type maintenance activity;
- comparing the initial digital image (Di) with the later digital image (Dx) to identify a presence of an object projecting from the tube end,
**characterised in that**
each acquiring step comprises acquiring one pair of digital images of the tube end from two different vantage points.

2. The method of claim 1, wherein acquiring said pair of digital images of the tube end from two different viewing angles is performed using a catadioptric system comprising a single imaging device.

3. The method of any one of the foregoing claims, further comprising the step of creating a data record in a relational database for the tube end, said data record containing a value representing the presence of the identified object.

4. The method of any one of claims 1 or 2 further comprising the step of creating a data record in a relational database for a plurality of tube ends of the shell and tube device.

5. The method of either claim 3 or claim 4 further comprising the step of using one or more data records stored in the relational database to produce one or more of tables, graphs, spreadsheets, and color-coded summary graphics.

6. The method of any one of the foregoing claims further comprising the step of producing performance metrics for the projection-type maintenance activity, wherein the producing step comprises calculating and displaying the performance metrics in a table, graph, spreadsheet, or color-coded summary graphic.

7. The method of claim 6 further comprising displaying said performance metrics on a mobile visual display device.

8. The method of either claim 3 or claim 4, further comprising the steps of:
- measuring one or more workspace parameters,
- recording workspace parameter measurements in the relational database, and
- optionally, presenting said workspace parameter measurements on a visual display.

9. The method of any one of the foregoing claims, wherein said shell and tube device is one of a reactor, preheater, boiler, superheater, reboiler, condenser, evaporator, recuperator, Quench Exchanger, Transfer Line Exchanger (TLE) and cross-exchanger.

10. A method for monitoring the status of
a shell and tube device comprising a tube sheet including a plurality of tube ends arranged in a fixed pattern of rows and columns, being
a method for monitoring the status of the shell and tube device during a projection-type maintenance activity, **characterized in that** said method comprises:
a) assigning a unique identifier to each of the tube ends,
b) at an initial acquisition time (Ti), acquiring one pair of initial digital images (Dai and Dbi) of at least a portion of the tube sheet from two different vantage points,
c) determining an initial state of an attribute (Ai) having at least two states for each of the tube ends within said pair of initial digital images (Dai and Dbi), and
d) creating an initial data record in a relational database for each tube end within said pair of initial digital images (Dai and Dbi), said initial data record including:
i. the initial acquisition time (Ti),
ii. the unique identifier for the tube end, and
iii. an initial state of the image attribute (Ai) at the initial acquisition time (Ti).

11. The method of claim 10, further comprising:
e) at a later acquisition time (Tx), wherein Tx > Ti, acquiring a later pair of digital images (Dax and Dbx) of at least a portion of the tube sheet from two different vantage points,
f) determining a later state of the attribute (Ax) for each tube end in said later pair of digital images (Dax and Dbx), and
g) creating a later data record in the relational database for each tube end within said later pair of digital images (Dax and Dbx), said later data record including:
i. the later acquisition time (Tx),
ii. the unique identifier for the tube end,
iii. the later state of the selected attribute (Ax) at the later acquisition time (Tx), and
h) repeating steps e) though g) until said projection-type maintenance activity is complete.

12. The method of either claim 10 or claim 11, wherein said shell and tube device is one of a reactor, preheater, boiler, superheater, reboiler, condenser, evaporator, recuperator, Quench Exchanger, Transfer Line Exchanger (TLE) and cross-exchanger.

13. The method of any one of claims 10-12, wherein said shell and tube device is a reactor utilized to perform a chemical conversion, said chemical conversion comprising:
i. conversion of propylene to acrolein and/or acrylic acid;
ii. conversion of propane to acrolein and/or acrylic acid;
iii. conversion of glycerol to acrolein and/or acrylic acid;
iv. conversion of tert-butanol, isobutene, isobutane, isobutyraldehyde, isobutyric acid, or methyl tert-butyl ether to methacrolein and/or methacrylic acid;
v. conversion of acrolein to acrylic acid;
vi. conversion of methacrolein to methacrylic acid;
vii. conversion of o-xylene or naphthalene to phthalic anhydride;
viii. conversion of butadiene to maleic anhydride;
ix. conversion of n-butane to maleic anhydride;
x. conversion of indanes to anthraquinone;
xi. conversion of ethylene to ethylene oxide; or
xii. conversion of propylene to propylene oxide.

14. The method of any one of claims 10-13 further comprising the step of using one or more data records stored in the relational database to produce one or more of tables, graphs, spreadsheets, and color-coded summary graphics.

15. The method of any one of claims 10-14 further comprising the step of producing performance metrics for the projection-type maintenance activity, wherein the producing step comprises calculating and displaying the performance metrics in a table, graph, spreadsheet, or color-coded summary graphic.

16. The method of claim 15 further comprising displaying said performance metrics on a mobile visual display device.

17. The method of any one of claims 10-16, further comprising the steps of:
- measuring one or more workspace parameters,
- recording workspace parameter measurements in the relational database, and
- optionally, presenting said workspace parameter measurements on a visual display.

18. A method for monitoring the status of
a shell and tube device comprising a tube sheet including a plurality of tube ends arranged in a fixed pattern of rows and columns, being
a method for monitoring the status of the shell and tube device during a projection-type maintenance activity, **characterized in that** said method comprises:
a) assigning a unique identifier to each of the tube ends,
b) at an initial acquisition time (Ti), acquiring at least one pair of initial digital images (Dai and Dbi) of at least a portion of the tube sheet from two different vantage points,
c) processing the at least one pair of initial digital images to create an initial Enhanced Digital Image (Ei),
d) determining an initial state of an attribute (Ai) having at least two states for each of the tube ends within said initial Enhanced digital image (Ei), and
e) creating an initial data record in a relational database for each tube end within said initial Enhanced digital image (Ei), said initial data record including:
i. the initial acquisition time (Ti),
ii. the unique identifier for the tube end, and
iii. an initial state of the image attribute (Ai) at the initial acquisition time (Ti).

19. The method of claim 18, further comprising:
f) at a later acquisition time (Tx), wherein Tx > Ti, acquiring at least one pair of later digital images (Dax and Dbx) of at least a portion of the tube sheet from two different vantage points,
g) processing the at least one pair of later digital images to create a later Enhanced Digital Image (Ex),
h) determining a later state of an attribute (Ax) for each tube end in said later Enhanced digital image (Ex), and
i) creating a later data record in the relational database for each tube end within said later Enhanced digital image (Ex), said later data record including:
i. the later acquisition time (Tx),
ii. the unique identifier for the tube end,
iii. the later state of the selected attribute (Ax) at the later acquisition time (Tx), and
j) repeating steps f) though i) until said projection-type maintenance activity is complete.

20. The method of either claim 18 or claim 19, wherein said shell and tube device is one of a reactor, preheater, boiler, superheater, reboiler, condenser, evaporator, recuperator, Quench Exchanger, Transfer Line Exchanger (TLE) and cross-exchanger.

21. The method of any one of claims 18-20 further comprising the step of using one or more data records stored in the relational database to produce one or more of tables, graphs, spreadsheets, and color-coded summary graphics.

22. The method of any one of claims 18-21 further comprising the step of producing performance metrics for the projection-type maintenance activity, wherein the producing step comprises calculating and displaying the performance metrics in a table, graph, spreadsheet, or color-coded summary graphic.

23. The method of claim 22 further comprising displaying said performance metrics on a mobile visual display device.

## Patentansprüche

1. Verfahren zur Überwachung eines Status einer Rohrbündelvorrichtung während einer Vorsprungswartungsaktivität, wobei das Verfahren Folgendes umfasst:
- Erfassen eines anfänglichen digitalen Bildes (Di) eines Rohrendes auf mindestens einem Abschnitt eines Rohrbodens der Rohrbündelvorrichtung;
- Erfassen eines späteren digitalen Bildes (Dx) des Rohrendes zu einem späteren Zeitpunkt während der Vorsprungswartungsaktivität;
- Vergleichen des anfänglichen digitalen Bildes (Di) mit dem späteren digitalen Bild (Dx), um das Vorhandensein eines Objekts zu identifizieren, das vom Rohrende vorspringt;
**dadurch gekennzeichnet, dass** jeder Erfassungsschritt das Erfassen eines Paars digitaler Bilder des Rohrendes aus zwei verschiedenen Blickwinkeln umfasst.

2. Verfahren nach Anspruch 1, wobei das Erfassen des Paars digitaler Bilder des Rohrendes aus zwei verschiedenen Blickwinkeln unter Verwendung eines katadioptrischen Systems erfolgt, das eine einzige Bildgebungsvorrichtung umfasst.

3. Verfahren nach einem der vorhergehenden Ansprüche, ferner umfassend den Schritt des Erstellens eines Datensatzes in einer relationalen Datenbank für das Rohrende, wobei der Datensatz einen Wert enthält, der das Vorhandensein des identifizierten Objekts darstellt.

4. Verfahren nach einem der Ansprüche 1 oder 2, ferner umfassend den Schritt des Erstellens eines Datensatzes in einer relationalen Datenbank für eine Mehrzahl von Rohrenden der Rohrbündelvorrichtung.

5. Verfahren nach Anspruch 3 oder Anspruch 4, ferner umfassend den Schritt des Verwendens eines oder mehrerer Datensätze, die in der relationalen Datenbank gespeichert sind, um eines oder mehrere von Tabellen, Graphen, Spreadsheets und farbcodierten Übersichtsgrafiken zu erzeugen.

6. Verfahren nach einem der vorhergehenden Ansprüche, ferner umfassend den Schritt des Erzeugens von Leistungsmetriken für die Vorsprungswartungsaktivität, wobei der Erzeugungsschritt Berechnen und Anzeigen der Leistungsmetriken in einer Tabelle, einem Graphen, einem Spreadsheet oder einer farbcodierten Übersichtsgrafik umfasst.

7. Verfahren nach Anspruch 6, ferner umfassend Anzeigen der Leistungsmetriken auf einer mobilen visuellen Anzeigevorrichtung.

8. Verfahren nach Anspruch 3 oder Anspruch 4, ferner umfassend die folgenden Schritte:
- Messen eines oder mehrerer Workspace-Parameter,
- Aufzeichnen von Workspace-Parametermessungen in der relationalen Datenbank, und
- optionales Darstellen der Workspace-Parametermessungen auf einer visuellen Anzeige.

9. Verfahren nach einem der vorhergehenden Ansprüche, wobei die Rohrbündelvorrichtung eines von einem Reaktor, einem Vorwärmer, einem Boiler, einem Überhitzer, einem Aufkocher, einem Kondensator, einem Verdampfer, einem Rekuperator, einem Quenchkühler, einem Spaltgaskühler (TLE) und einem Kreuzstrom-Wärmetauscher ist.

10. Verfahren zur Überwachung des Status einer Rohrbündelvorrichtung, die einen Rohrboden mit einer Mehrzahl von Rohrenden umfasst, die in einem festen Muster von Reihen und Spalten angeordnet sind, wobei es sich um ein Verfahren zur Überwachung des Status der Rohrbündelvorrichtung während einer Vorsprungswartungsaktivität handelt,
**dadurch gekennzeichnet, dass** das Verfahren Folgendes umfasst:
a) Zuweisen einer eindeutigen Kennung zu jedem der Rohrenden,
b) zu einem anfänglichen Erfassungszeitpunkt (Ti) Erfassen eines Paars anfänglicher digitaler Bilder (Dai und Dbi) mindestens eines Abschnitts des Rohrbodens aus zwei verschiedenen Blickwinkeln,
c) Bestimmen eines anfänglichen Zustands eines Attributs (Ai) mit mindestens zwei Zuständen für jedes der Rohrenden innerhalb des Paars anfänglicher digitaler Bilder (Dai und Dbi) und
d) Erstellen eines anfänglichen Datensatzes in einer relationalen Datenbank für jedes Rohrende innerhalb des Paares anfänglicher digitaler Bilder (Dai und Dbi), wobei der anfängliche Datensatz Folgendes beinhaltet:
i. den anfänglichen Erfassungszeitpunkt (Ti),
ii. die eindeutige Kennung für das Rohrende und
iii. einen anfänglichen Zustand des Bildattributs (Ai) zum anfänglichen Erfassungszeitpunkt (Ti).

11. Verfahren nach Anspruch 10, ferner umfassend:
e) zu einem späteren Erfassungszeitpunkt (Tx), wobei Tx > Ti, Erfassen eines späteren Paars digitaler Bilder (Dax und Dbx) mindestens eines Abschnitts des Rohrbodens aus zwei verschiedenen Blickwinkeln,
f) Bestimmen eines späteren Zustands des Attributs (Ax) für jedes Rohrende in dem späteren Paar digitaler Bilder (Dax und Dbx) und
g) Erstellen eines späteren Datensatzes in der relationalen Datenbank für jedes Rohrende innerhalb des späteren Paars digitaler Bilder (Dax und Dbx), wobei der spätere Datensatz Folgendes beinhaltet:
i. den späteren Erfassungszeitpunkt (Tx),
ii. die eindeutige Kennung für das Rohrende,
iii. den späteren Zustand des ausgewählten Attributs (Ax) zum späteren Erfassungszeitpunkt (Tx) und
h) Wiederholen der Schritte e) bis g), bis die Vorsprungswartungsaktivität abgeschlossen ist.

12. Verfahren nach Anspruch 10 oder Anspruch 11, wobei die Rohrbündelvorrichtung eines von einem Reaktor, einem Vorwärmer, einem Boiler, einem Überhitzer, einem Aufkocher, einem Kondensator, einem Verdampfer, einem Rekuperator, einem Quenchkühler, einem Spaltgaskühler (TLE) und einem Kreuzstrom-Wärmetauscher ist.

13. Verfahren nach einem der Ansprüche 10-12, wobei die Rohrbündelvorrichtung ein Reaktor ist, der verwendet wird, um eine chemische Umwandlung durchzuführen, wobei die chemische Umwandlung folgende umfasst:
i. Umwandlung von Propylen in Acrolein und/oder Acrylsäure;
ii. Umwandlung von Propan in Acrolein und/oder Acrylsäure;
iii. Umwandlung von Glycerin in Acrolein und/oder Acrylsäure;
iv. Umwandlung von tert-Butanol, Isobuten, Isobutan, Isobutyraldehyd, Isobuttersäure oder Methyl-tert-butylether in Methacrolein und/oder Methacrylsäure;
v. Umwandlung von Acrolein in Acrylsäure;
vi. Umwandlung von Methacrolein in Methacrylsäure;
vii. Umwandlung von o-Xylol oder Naphthalin in Phthalsäureanhydrid;
viii. Umwandlung von Butadien in Maleinsäureanhydrid;
ix. Umwandlung von n-Butan in Maleinsäureanhydrid;
x. Umwandlung von Indanen in Anthrachinon;
xi. Umwandlung von Ethylen in Ethylenoxid oder
xii. Umwandlung von Propylen in Propylenoxid.

14. Verfahren nach einem der Ansprüche 10-13, ferner umfassend den Schritt des Verwendens eines oder mehrerer Datensätze, die in der relationalen Datenbank gespeichert sind, um eines oder mehrere von Tabellen, Graphen, Spreadsheets und farbcodierten Übersichtsgrafiken zu erzeugen.

15. Verfahren nach einem der Ansprüche 10-14, ferner umfassend den Schritt des Erzeugens von Leistungsmetriken für die Vorsprungswartungsaktivität, wobei der Erzeugungsschritt Berechnen und Anzeigen der Leistungsmetriken in einer Tabelle, einem Graphen, einem Spreadsheet oder einer farbcodierten Übersichtsgrafik umfasst.

16. Verfahren nach Anspruch 15, ferner umfassend Anzeigen der Leistungsmetriken auf einer mobilen visuellen Anzeigevorrichtung.

17. Verfahren nach einem der Ansprüche 10-16, ferner umfassend die folgenden Schritte:
- Messen eines oder mehrerer Workspace-Parameter,
- Aufzeichnen von Workspace-Parametermessungen in der relationalen Datenbank, und
- optionales Darstellen der Workspace-Parametermessungen auf einer visuellen Anzeige.

18. Verfahren zur Überwachung des Status einer Rohrbündelvorrichtung, die einen Rohrboden mit einer Mehrzahl von Rohrenden umfasst, die in einem festen Muster von Reihen und Spalten angeordnet sind, wobei es sich um ein Verfahren zur Überwachung des Status der Rohrbündelvorrichtung während einer Vorsprungswartungsaktivität handelt,
**dadurch gekennzeichnet, dass** das Verfahren Folgendes umfasst:
a) Zuweisen einer eindeutigen Kennung zu jedem der Rohrenden,
b) zu einem anfänglichen Erfassungszeitpunkt (Ti) Erfassen eines Paars anfänglicher digitaler Bilder (Dai und Dbi) mindestens eines Abschnitts des Rohrbodens aus zwei verschiedenen Blickwinkeln,
c) Verarbeiten des mindestens einen Paars anfänglicher digitaler Bilder, um ein anfängliches verbessertes digitales Bild (Ei) zu erstellen,
d) Bestimmen eines anfänglichen Zustands eines Attributs (Ai) mit mindestens zwei Zuständen für jedes der Rohrenden innerhalb des anfänglichen verbesserten digitalen Bildes (Ei) und
e) Erstellen eines anfänglichen Datensatzes in einer relationalen Datenbank für jedes Rohrende innerhalb des anfänglichen verbesserten digitalen Bildes (Ei), wobei der anfängliche Datensatz Folgendes beinhaltet:
i. den anfänglichen Erfassungszeitpunkt (Ti),
ii. die eindeutige Kennung für das Rohrende und
iii. einen anfänglichen Zustand des Bildattributs (Ai) zum anfänglichen Erfassungszeitpunkt (Ti).

19. Verfahren nach Anspruch 18, ferner umfassend:
f) zu einem späteren Erfassungszeitpunkt (Tx), wobei Tx > Ti, Erfassen mindestens eines Paars späterer digitaler Bilder (Dax und Dbx) mindestens eines Abschnitts des Rohrbodens aus zwei verschiedenen Blickwinkeln,
g) Verarbeiten des mindestens einen Paars späterer digitaler Bilder, um ein späteres verbessertes digitales Bild (Ex) zu erstellen,
h) Bestimmen eines späteren Zustands eines Attributs (Ax) für jedes Rohrende in dem späteren verbesserten digitalen Bild (Ex) und
i) Erstellen eines späteren Datensatzes in der relationalen Datenbank für jedes Rohrende innerhalb des späteren verbesserten digitalen Bildes (Ex), wobei der spätere Datensatz Folgendes beinhaltet:
i. den späteren Erfassungszeitpunkt (Tx),
ii. die eindeutige Kennung für das Rohrende,
iii. den späteren Zustand des ausgewählten Attributs (Ax) zum späteren Erfassungszeitpunkt (Tx) und
j) Wiederholen der Schritte f) bis i), bis die Vorsprungswartungsaktivität abgeschlossen ist.

20. Verfahren nach Anspruch 18 oder Anspruch 19, wobei die Rohrbündelvorrichtung eines von einem Reaktor, einem Vorwärmer, einem Boiler, einem Überhitzer, einem Aufkocher, einem Kondensator, einem Verdampfer, einem Rekuperator, einem Quenchkühler, einem Spaltgaskühler (TLE) und einem Kreuzstrom-Wärmetauscher ist.

21. Verfahren nach einem der Ansprüche 18-20, ferner umfassend den Schritt des Verwendens eines oder mehrerer Datensätze, die in der relationalen Datenbank gespeichert sind, um eines oder mehrere von Tabellen, Graphen, Spreadsheets und farbcodierten Übersichtsgrafiken zu erzeugen.

22. Verfahren nach einem der Ansprüche 18-21, ferner umfassend den Schritt des Erzeugens von Leistungsmetriken für die Vorsprungswartungsaktivität, wobei der Erzeugungsschritt Berechnen und Anzeigen der Leistungsmetriken in einer Tabelle, einem Graphen, einem Spreadsheet oder einer farbcodierten Übersichtsgrafik umfasst.

23. Verfahren nach Anspruch 22, ferner umfassend Anzeigen der Leistungsmetriken auf einer mobilen visuellen Anzeigevorrichtung.

## Revendications

1. Procédé pour surveiller un statut d'un dispositif multitubulaire à calandre durant une activité de maintenance de type saillie, dans lequel ledit procédé comprend les faits :
- d'acquérir une image numérique initiale (Di) d'une extrémité de tube sur au moins une partie d'une plaque tubulaire du dispositif multitubulaire à calandre ;
- d'acquérir une image numérique ultérieure (Dx) de l'extrémité de tube à un temps ultérieur durant l'activité de maintenance de type saillie ;
- de comparer l'image numérique initiale (Di) à l'image numérique ultérieure (Dx) pour identifier une présence d'un objet faisant saillie à partir de l'extrémité de tube,
**caractérisé en ce que** chaque étape consistant à acquérir comprend le fait d'acquérir une paire d'images numériques de l'extrémité de tube depuis deux points d'observation différents.

2. Procédé de la revendication 1, dans lequel le fait d'acquérir ladite paire d'images numériques de l'extrémité de tube depuis deux angles de vue différents est réalisé en utilisant un système catadioptrique comprenant un seul dispositif d'imagerie.

3. Procédé de l'une quelconque des revendications précédentes, comprenant en outre l'étape consistant à créer un enregistrement de données dans une base de données relationnelle pour l'extrémité de tube, ledit enregistrement de données contenant une valeur représentant la présence de l'objet identifié.

4. Procédé de l'une quelconque des revendications 1 ou 2, comprenant en outre l'étape consistant à créer un enregistrement de données dans une base de données relationnelle pour une pluralité d'extrémité de tubes du dispositif multitubulaire à calandre.

5. Procédé de l'une ou l'autre de la revendication 3 ou de la revendication 4, comprenant en outre l'étape consistant à utiliser un ou plusieurs enregistrements de données stockés dans la base de données relationnelle pour produire un ou plusieurs parmi des tables, des graphes, des tableurs, et des graphiques résumés codés couleur.

6. Procédé de l'une quelconque des revendications précédentes, comprenant en outre l'étape consistant à produire des mesures de performance pour l'activité de maintenance de type saillie, dans lequel l'étape consistant à produire comprend les faits de calculer et d'afficher les mesures de performance dans une table, un graphe, un tableur, ou un graphique résumé codé couleur.

7. Procédé de la revendication 6, comprenant en outre le fait d'afficher lesdites mesures de performance sur un dispositif d'affichage visuel mobile.

8. Procédé de l'une ou l'autre de la revendication 3 ou de la revendication 4, comprenant en outre les étapes suivantes :
- mesurer un ou plusieurs paramètres d'espace de travail,
- enregistrer des mesures de paramètres d'espace de travail dans la base de données relationnelle, et
- facultativement, présenter lesdites mesures de paramètres d'espace de travail sur un écran d'affichage visuel.

9. Procédé de l'une quelconque des revendications précédentes, dans lequel ledit dispositif multitubulaire à calandre est un parmi un réacteur, un préchauffeur, une chaudière, un surchauffeur, un rebouilleur, un condenseur, un évaporateur, un récupérateur, un échangeur de trempe, un échangeur à ligne de transfert (Transfer Line Exchanger, TLE), et un échangeur croisé.

10. Procédé pour surveiller le statut d'un dispositif multitubulaire à calandre comprenant une plaque tubulaire incluant une pluralité d'extrémités de tube agencées en une configuration fixe de rangées et de colonnes, étant un procédé pour surveiller le statut du dispositif multitubulaire à calandre durant une activité de maintenance de type saillie,
**caractérisé en ce que** ledit procédé comprend les faits :
a) d'attribuer un identifiant unique à chacune des extrémités de tube,
b) à un temps d'acquisition initial (Ti), d'acquérir une paire d'images numériques initiales (Dai et Dbi) d'au moins une partie de la plaque tubulaire depuis deux points d'observation différents,
c) de déterminer un état initial d'un attribut (Ai) ayant au moins deux états pour chacune des extrémités de tube à l'intérieur de ladite paire d'images numériques initiales (Dai et Dbi), et
d) de créer un enregistrement de données initial dans une base de données relationnelle pour chaque extrémité de tube à l'intérieur de ladite paire d'images numériques initiales (Dai et Dbi), ledit enregistrement de données initial incluant :
i. le temps d'acquisition initial (Ti),
ii. l'identifiant unique pour l'extrémité de tube, et
iii. un état initial de l'attribut d'image (Ai) au temps d'acquisition initial (Ti).

11. Procédé de la revendication 10, comprenant en outre les faits :
e) à un temps d'acquisition ultérieur (Tx), dans lequel Tx > Ti, d'acquérir une paire ultérieure d'images numériques (Dax et Dbx) d'au moins une partie de la plaque tubulaire depuis deux points d'observation différents,
f) de déterminer un état ultérieur de l'attribut (Ax) pour chaque extrémité de tube dans ladite paire ultérieure d'images numériques (Dax et Dbx), et
g) de créer un enregistrement de données ultérieur dans la base de données relationnelle pour chaque extrémité de tube à l'intérieur de ladite paire ultérieure d'images numériques (Dax et Dbx), ledit enregistrement de données ultérieur incluant :
i. le temps d'acquisition ultérieur (Tx),
ii. l'identifiant unique pour l'extrémité de tube,
iii. l'état ultérieur de l'attribut sélectionné (Ax) au temps d'acquisition ultérieur (Tx), et
h) de répéter les étapes e) à g) jusqu'à ce que ladite activité de maintenance de type saillie soit achevée.

12. Procédé de l'une ou l'autre de la revendication 10 ou de la revendication 11, dans lequel ledit dispositif multitubulaire à calandre est un parmi un réacteur, un préchauffeur, une chaudière, un surchauffeur, un rebouilleur, un condenseur, un évaporateur, un récupérateur, un échangeur de trempe, un échangeur à ligne de transfert (Transfer Line Exchanger, TLE), et un échangeur croisé.

13. Procédé de l'une quelconque des revendications 10 à 12, dans lequel ledit dispositif multitubulaire à calandre est un réacteur utilisé pour réaliser une conversion chimique, ladite conversion chimique comprenant :
i. conversion de propylène en acroléine et/ou acide acrylique ;
ii. conversion de propane en acroléine et/ou acide acrylique ;
iii. conversion de glycérol en acroléine et/ou acide acrylique ;
iv. conversion de tert-butanol, d'isobutène, d'isobutane, d'isobutyraldéhyde, d'acide isobutyrique, ou d'éther tert-butylméthylique en méthacroléine et/ou acide méthacrylique ;
v. conversion d'acroléine en acide acrylique ;
vi. conversion de méthacroléine en acide méthacrylique ;
vii. conversion de o-xylène ou de naphtalène en anhydride phtalique ;
viii. conversion de butadiène en anhydride maléique ;
ix. conversion de n-butane en anhydride maléique ;
x. conversion d'indanes en anthraquinone ;
xi. conversion d'éthylène en oxyde d'éthylène ; ou
xii. conversion de propylène en oxyde de propylène.

14. Procédé de l'une quelconque des revendications 10 à 13, comprenant en outre l'étape consistant à utiliser un ou plusieurs enregistrements de données stockés dans la base de données relationnelle pour produire un ou plusieurs parmi des tables, des graphes, des tableurs, et des graphiques résumés codés couleur.

15. Procédé de l'une quelconque des revendications 10 à 14 comprenant en outre l'étape consistant à produire des mesures de performance pour l'activité de maintenance de type saillie, dans lequel l'étape consistant à produire comprend les faits de calculer et d'afficher les mesures de performance dans une table, un graphe, un tableur, ou un graphique résumé codé couleur.

16. Procédé de la revendication 15, comprenant en outre le fait d'afficher lesdites mesures de performance sur un dispositif d'affichage visuel mobile.

17. Procédé de l'une quelconque des revendications 10 à 16, comprenant en outre les étapes suivantes :
- mesurer un ou plusieurs paramètres d'espace de travail,
- enregistrer des mesures de paramètres d'espace de travail dans la base de données relationnelle, et
- facultativement, présenter lesdites mesures de paramètres d'espace de travail sur un écran d'affichage visuel.

18. Procédé pour surveiller le statut d'un dispositif multitubulaire à calandre comprenant une plaque tubulaire incluant une pluralité d'extrémités de tube agencées en une configuration fixe de rangées et de colonnes, étant un procédé pour surveiller le statut du dispositif multitubulaire à calandre durant une activité de maintenance de type saillie,
**caractérisé en ce que** ledit procédé comprend les faits :
a) d'attribuer un identifiant unique à chacune des extrémités de tube,
b) à un temps d'acquisition initial (Ti), d'acquérir au moins une paire d'images numériques initiales (Dai et Dbi) d'au moins une partie de la plaque tubulaire depuis deux points d'observation différents,
c) de traiter l'au moins une paire d'images numériques initiales pour créer une image numérique améliorée initiale (Ei),
d) de déterminer un état initial d'un attribut (Ai) ayant au moins deux états pour chacune des extrémités de tube à l'intérieur de ladite image numérique améliorée initiale (Ei), et
e) de créer un enregistrement de données initial dans une base de données relationnelle pour chaque extrémité de tube à l'intérieur de ladite image numérique améliorée initiale (Ei), ledit enregistrement de données initial incluant :
i. le temps d'acquisition initial (Ti),
ii. l'identifiant unique pour l'extrémité de tube, et
iii. un état initial de l'attribut d'image (Ai) au temps d'acquisition initial (Ti).

19. Procédé de la revendication 18, comprenant en outre les faits :
f) à un temps d'acquisition ultérieur (Tx), dans lequel Tx > Ti, d'acquérir au moins une paire d'images numériques ultérieures (Dax et Dbx) d'au moins une partie de la plaque tubulaire depuis deux points d'observation différents,
g) de traiter l'au moins une paire d'images numériques ultérieures pour créer une image numérique améliorée ultérieure (Ex),
h) de déterminer un état ultérieur d'un attribut (Ax) pour chaque extrémité de tube dans ladite image numérique améliorée ultérieure (Ex), et
i) de créer un enregistrement de données ultérieur dans la base de données relationnelle pour chaque extrémité de tube à l'intérieur de ladite image numérique améliorée ultérieure (Ex), ledit enregistrement de données ultérieur incluant :
i. le temps d'acquisition ultérieur (Tx),
ii. l'identifiant unique pour l'extrémité de tube,
iii. l'état ultérieur de l'attribut sélectionné (Ax) au temps d'acquisition ultérieur (Tx), et
j) de répéter les étapes f) à i) jusqu'à ce que ladite activité de maintenance de type saillie soit achevée.

20. Procédé de l'une ou l'autre de la revendication 18 ou de la revendication 19, dans lequel ledit dispositif multitubulaire à calandre est un parmi un réacteur, un préchauffeur, une chaudière, un surchauffeur, un rebouilleur, un condenseur, un évaporateur, un récupérateur, un échangeur de trempe, un échangeur à ligne de transfert (Transfer Line Exchanger, TLE), et un échangeur croisé.

21. Procédé de l'une quelconque des revendications 18 à 20, comprenant en outre l'étape consistant à utiliser un ou plusieurs enregistrements de données stockés dans la base de données relationnelle pour produire un ou plusieurs parmi des tables, des graphes, des tableurs, et des graphiques résumés codés couleur.

22. Procédé de l'une quelconque des revendications 18 à 21 comprenant en outre l'étape consistant à produire des mesures de performance pour l'activité de maintenance de type saillie, dans lequel l'étape consistant à produire comprend les faits de calculer et d'afficher les mesures de performance dans une table, un graphe, un tableur, ou un graphique résumé codé couleur.

23. Procédé de la revendication 22, comprenant en outre le fait d'afficher lesdites mesures de performance sur un dispositif d'affichage visuel mobile.
